# EUROPEAN PATENT APPLICATION

(11) **EP 4 667 059 A2**
(43) Date of publication of application: **24.12.2025**
(21) Application number: 25207172.5
(22) Date of filing: 27.05.2016
(51) Int. Cl.: A61P 37/02

(54) **METHOD FOR TREATING OR PREVENTING DIABETIC NEPHROPATHY**

(30) Priority: 28.05.2015 JP 2015109207
(62) Divisional of application: 16800124.6
(71) Applicant: Japan Tobacco Inc., Tokyo 105-6927 (JP)
(72) Inventor: FUKUI, Kenji, Takatsuki-shi, Osaka, 569-1125 (JP); SHINOZAKI, Yuichi, Takatsuki-shi, Osaka, 569-1125 (JP); INAGAKI, Koji, Takatsuki-shi, Osaka, 569-1125 (JP)
(74) Representative: Vossius & Partner Patentanwälte Rechtsanwälte mbB

(57) **Abstract**

The present invention provides a method for treating or preventing a renal injury-related disease such as diabetic nephropathy comprising administering to a mammal a therapeutically effective amount of 2-({[7-hydroxy-5-(2-phenylethyl)-[1,2,4]triazolo[1,5-a]pyridin-8-yl]carbonyl}amino)acetic acid or a pharmaceutically acceptable salt thereof, and a novel pharmaceutical use of the aforementioned compound such as a pharmaceutical composition for treating or preventing a renal injury-related disease and the like comprising the aforementioned compound or a pharmaceutically acceptable salt thereof.

## Description

### [Technical Field]

The present invention relates to a novel pharmaceutical use of 2-({[7-hydroxy-5-(2-phenylethyl)-[1,2,4]triazolo[1,5-a]pyridin-8-yl]carbonyl}amino)acetic acid (hereinafter indicated as Compound A). More particularly, the present invention relates to a method for treating or preventing a renal injury-related disease such as diabetic nephropathy and a method for suppressing tubulointerstitial fibrosis comprising administering to a mammal a therapeutically effective amount of Compound A or a pharmaceutically acceptable salt thereof. In addition, the present invention relates to a pharmaceutical composition for treating or preventing a renal injury-related disease and an agent for suppressing tubulointerstitial fibrosis comprising Compound A or a pharmaceutically acceptable salt thereof.

### [Background Art]

Compound A is shown by the following chemical structural formula:

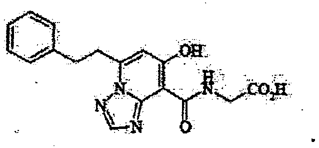

Compound A and a process thereof are reported in Patent Document 1.

Patent Document 1 describes that Compound A has a prolyl hydroxylase (PHD) inhibitory action and stabilizes the hypoxia-inducible factor (HIF); and that it is expected to improve diseases such as anemia including renal anemia (anemia associated with renal failure), ischemic cardiac diseases (angina pectoris, myocardial infarction etc.), ischemic cerebrovascular diseases (cerebral infarction, cerebral embolism, transient cerebral ischemic attack etc.), chronic renal failure (ischemic nephropathy, renal tubulointerstitial disorder etc.), diabetic complications (diabetic wound etc.), and cognitive impairment (dementia, Alzheimer's disease, Parkinson's disease, Huntington's disease etc.) by inhibiting PHD and stabilizing HIF.

Unlike the conventional disease name system of kidney diseases (diabetic nephropathy, chronic renal failure etc.), chronic kidney disease (CKD) is a concept including a wide range of pathology and syndrome, which has been introduced comparatively recently, and the purpose of the concept is to suppress the transition to end-stage renal disease (ESRD) and the onset of cardiovascular disease (Non-Patent Document 1). CKD is defined as a disease in which the following (i), (ii), or (iii) lasts for 3 months or more: (i) the presence of a renal injury represented by proteinuria (including microalbuminuria), (ii) a renal impairment with a glomerular filtration rate (GFR) of less than 60 mL/min/1.73 m², or (iii) both (i) and (ii). While GFR is accurately obtained from the measured values of inulin clearance or creatinine clearance, estimated GFR (eGFR) based on serum creatinine value or serum cystatin C value can also be used as GFR. The main causes of CKD include, in addition to diabetic nephropathy, hypertensive nephrosclerosis, and primary and secondary glomerulopathies (including glomerulonephritis).

In recent years, it has been clarified that the prognosis of CKD and decline in GFR correlate well with tubulointerstitial lesions rather than glomerular lesions, and it has been recognized that the main locus of a final common pathway leading to end-stage renal disease is the tubulointerstitium (Non-Patent Documents 2, 3 and 4). The kidney, regardless of its abundant blood flow, shows poor uptake efficiency of oxygen, and especially the renal tubule is vulnerable to hypoxia because the renal tubule has a high consumption of oxygen and a high demand for oxygen (Non-Patent Documents 3 and 4). It has been shown that hypoxia, fibrosis and renal impairment progress at the same time in the kidney of CKD patients (Non-Patent Document 5). That is, as a final common pathway leading to end-stage renal disease caused by the progression of renal impairment, researchers have proposed a vicious circle in which hypoxic condition causes a deterioration of tubulointerstitial lesions and thereby progressing fibrosis, a decrease in capillary vessels causes a decrease in oxygen diffusion capacity as well as in oxygen supply, and then the kidney further becomes hypoxia (Non-Patent Document 4).

Diabetic nephropathy is caused by glomerular sclerosis and fibrosis which are due to changes of metabolism and hemodynamics in diabetes, and diabetic nephropathy is also one of the common causes of nephrotic syndrome and end-stage renal disease. Diabetic nephropathy is also one of the representative causes of CKD as described above. In diabetic nephropathy, GFR is increased (i.e., hyperfiltration is observed) at the time of onset, normalized along with early renal injury and mild hypertension, and then decreased over time. Subsequently, 30 to 300 mg/day of albumin, which is undetectable in routine urinalysis, is excreted in the urine (i.e., excretion of microalbuminuria). Thereafter, excretion of albumin is increased, and microalbuminuria becomes proteinuria of more than 0.5 g/day. Diabetic nephropathy at these early stages is asymptomatic, and the earliest warning is often the proteinuria detectable by routine urinalysis.

It has been conventionally known that there is a correlation between tubulointerstitial fibrosis and renal impairment in patients with diabetic nephropathy. Therefore, it is likely that in patients with diabetic nephropathy, further progression of renal injury leads the patients to the above-mentioned final common pathway (i.e., exacerbation and fibrosis of tubulointerstitial lesions due to hypoxia), thereby decreasing GFR and causing end-stage renal disease.

SHR/NDmcr-cp (cp/cp) rat is an animal model showing spontaneous hypertension; and showing metabolic abnormality similar to that of human type 2 diabetes such as overeating and obesity associated with overeating, hyperglycemia, hyperlipidemia, and hyperinsulinemia (Non-Patent Document 7). SHR/NDmcr-cp rat at 27 weeks of age does not show a change in plasma creatinine concentration; however, it shows a marked elevation of protein in urine (i.e., proteinuria), and has a histologically-evident glomerular disorder and tubulointerstitial damage which are consistent with the characteristics of diabetic nephropathy (Non-Patent Document 7).

### [Document List]

### [Patent Document]

Patent Document 1: WO 2011/007856

### [Non-Patent Documents]

Non-Patent Document 1: The Japanese Society of Nephrology ed., CKD clinical practice guidelines based on evidence 2009.
Non-Patent Document 2: Nangaku, M. and Eckardt, K. "Hypoxia and the HIF system in kidney disease." J. Mol. Med. (2007) Vol. 85, pages 1325-1330.
Non-Patent Document 3: Nangaku, M. et al. "Hypoxia and hypoxia-inducible factor inrenal disease." Nephron Exp. Nephrol. (2008) Vol. 110, pages e1-e7.
Non-Patent Document 4: Masaomi Nangaku, Final common pathway of kidney disease: hypoxic disorder", Journal of Japanese Society of Pediatric Nephrology, Vol. 25 (2012), pages 132-136.
Non-Patent Document 5: Inoue, T. et al. "Noninvasive evaluation of kidney hypoxia and fibrosis using magnetic resonance imaging." J. Am. Soc. Nephrol. (2011) Vol. 22, pages 1429-1434.
Non-Patent Document 6: Taft, J.L. et al. "Clinical and histological correlations of decline in renal function in diabetic patients with proteinuria." Diabetes (1994) Vol. 43, pages 1046-1051.
Non-Patent Document 7: Nangaku, M. et al. "In a type 2 diabetic nephropathy rat model, the improvement of obesity by a low calorie diet reduces oxidative/carbonyl stress and prevents diabetic nephropathy." Nephrol. Dial. Transplant. (2005) Vol. 20, pages 2661-2669.

### [SUMMARY OF THE INVENTION]

### [Problems to be Solved by the Invention]

The problem to be solved by the invention is to provide a novel pharmaceutical use of Compound A.

### [Means of Solving the Problems]

The present inventors have found that Compound A suppresses a tubulointerstitial fibrosis induced by local hypoxic condition, and a tubulointerstitial fibrosis induced by hyperglycemic condition. In addition, based on the finding that Compound A suppresses a tubulointerstitial fibrosis caused by hypoxia, the present inventors have found that Compound A is effective for diabetic nephropathy and a chronic kidney disease associated with diabetes.

Accordingly, the present invention provides the following.
[1] A method for treating or preventing diabetic nephropathy comprising administering to a mammal a therapeutically effective amount of a compound represented by the following chemical structural formula: 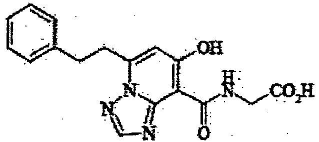 or a pharmaceutically acceptable salt thereof.
[2] A method for treating or preventing a chronic kidney disease associated with diabetes comprising administering to a mammal a therapeutically effective amount of a compound represented by the following chemical structural formula: 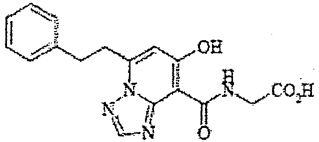 or a pharmaceutically acceptable salt thereof.
[3] A method for treating or preventing a chronic kidney disease caused by hypoxia which is selected from the group consisting of a chronic kidney disease associated with an immune system or inflammatory disease and a chronic kidney disease associated with hypertension comprising administering to a mammal a therapeutically effective amount of a compound represented by the following chemical structural formula: 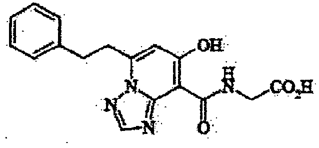 or a pharmaceutically acceptable salt thereof.
[4] The method according to [3] wherein the chronic kidney disease caused by hypoxia is a chronic kidney disease associated with an immune system or inflammatory disease.
[5] The method according to [3] wherein the chronic kidney disease caused by hypoxia is a chronic kidney disease associated with hypertension.
[6] A method for treating or preventing glomerulonephritis comprising administering to a mammal a therapeutically effective amount of a compound represented by the following chemical structural formula: 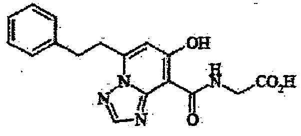 or a pharmaceutically acceptable salt thereof.
[7] A method for treating or preventing nephrosclerosis comprising administering to a mammal a therapeutically effective amount of a compound represented by the following chemical structural formula: 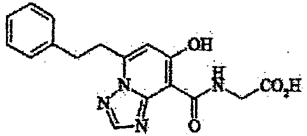 or a pharmaceutically acceptable salt thereof.
[8] A method for suppressing tubulointerstitial fibrosis comprising administering to a mammal a therapeutically effective amount of a compound represented by the following chemical structural formula: 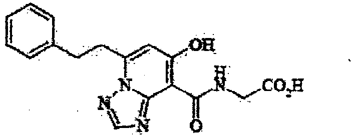 or a pharmaceutically acceptable salt thereof.
[9] A pharmaceutical composition for treating or preventing a renal injury-related disease selected from the group consisting of diabetic nephropathy, a chronic kidney disease associated with diabetes, glomerulonephritis, a chronic kidney disease associated with an immune system or inflammatory disease, nephrosclerosis, and a chronic kidney disease associated with hypertension comprising a compound represented by the following chemical structural formula: 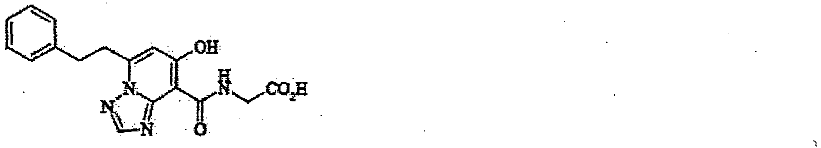 or a pharmaceutically acceptable salt thereof.
[10] An agent for suppressing tubulointerstitial fibrosis comprising a compound represented by the following chemical structural formula: 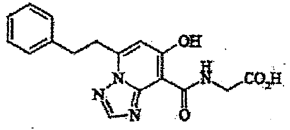 or a pharmaceutically acceptable salt thereof.
[11] Use of a compound represented by the following chemical structural formula: 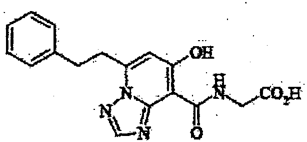 or a pharmaceutically acceptable salt thereof for the manufacture of a medicament for treating or preventing a renal injury-related disease selected from the group consisting of diabetic nephropathy, a chronic kidney disease associated with diabetes, glomerulonephritis, a chronic kidney disease associated with an immune system or inflammatory disease, nephrosclerosis, and a chronic kidney disease associated with hypertension.
[12] Use of a compound represented by the following chemical structural formula: 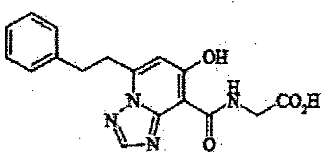 or a pharmaceutically acceptable salt thereof for the manufacture of an agent for suppressing tubulointerstitial fibrosis.

### [Effect of the Invention]

Compound A suppresses a tubulointerstitial fibrosis induced by hyperglycemic condition or local hypoxic condition, and it can thus be used for treating or preventing various renal injury-related diseases such as diabetic nephropathy, a chronic kidney disease associated with diabetes, and other chronic kidney diseases caused by hypoxia.

### [Brief Description of the Drawings]

Fig. 1 shows a suppressive effect on tubulointerstitial fibrosis by dietary administration of Compound A in a rat model of diabetic nephropathy at 12 weeks of age for 28 weeks (Example 1). The vertical axis shows a sirius red-stained area ratio (%).
Fig. 2 shows representative stained images showing a suppressive effect on tubulointerstitial fibrosis by dietary administration of Compound A in a rat model of diabetic nephropathy at 12 weeks of age for 28 weeks (Example 1).
Fig. 3 shows a suppressive effect on tubulointerstitial fibrosis by repeated oral administration of Compound A in a rat model of renal ischemia-reperfusion injury after 4 weeks from the ischemia-reperfusion injury, wherein Compound A was administered for 6 days starting from 3 days before the ischemia-reperfusion injury (Example 2). The vertical axis shows a sirius red-stained area ratio (%).

### [Description of Embodiments]

The definitions of terms in the present specification are as follows.

Compound A is 2-({[7-hydroxy-5-(2-phenylethyl)-[1,2,4]triazolo[1,5-a]pyridin-8-yl]carbonyl}amino)acetic acid), which is represented by the following chemical structural formula: 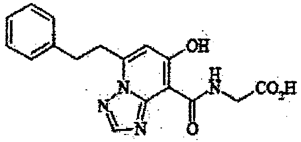 and can be prepared by a method known per se, for example, the method described in Patent Document 1.

The "pharmaceutically acceptable salt" may be any salt as long as it can form a nontoxic salt with Compound A; and means that, for example, Compound A can form salts with inorganic acids, organic acids, inorganic bases, organic bases, amino acids and the like.

Examples of the salts with inorganic acids include salts with hydrochloric acid, nitric acid, sulfuric acid, phosphoric acid, hydrobromic acid and the like.

Examples of the salts with organic acids include salts with oxalic acid, maleic acid, citric acid, fumaric acid, lactic acid, malic acid, succinic acid, tartaric acid, acetic acid, trifluoroacetic acid, gluconic acid, ascorbic acid, methanesulfonic acid, benzenesulfonic acid, p-toluenesulfonic acid and the like.

Examples of the salts with inorganic bases include sodium salt, potassium salt, calcium salt, magnesium salt, and ammonium salt.

Examples of the salts with organic bases include salts with methylamine, diethylamine, trimethylamine, triethylamine, ethanolamine, diethanolamine, triethanolamine, ethylenediamine, tris(hydroxymethyl)methylamine, dicyclohexylamine, N,N'-dibenzylethylenediamine, guanidine, pyridine, picoline, choline, cinchonine, meglumine and the like.

Examples of the salts with amino acids include salts with lysine, arginine, aspartic acid, glutamic acid and the like.

According to a known method, Compound A can be reacted with an inorganic base, an organic base, an inorganic acid, an organic acid or an amino acid to obtain each of the corresponding salts.

Among Compound A and a pharmaceutically acceptable salt thereof, Compound A is preferable.

The "pharmaceutical composition" means a composition which can be used as a pharmaceutical product.

The pharmaceutical composition for treating or preventing a renal injury-related disease of the present invention is prepared according to a method known in the technical field of pharmaceutical preparation by appropriately mixing Compound A or a pharmaceutically acceptable salt thereof with an appropriate amount of at least one type of pharmaceutically acceptable carrier and the like. The amount of Compound A or a pharmaceutically acceptable salt thereof contained in the pharmaceutical composition varies depending on the dosage form, dose and the like.

The "pharmaceutical composition" of the present invention can be administered orally or parenterally. The administration form includes oral administration; and parenteral administration such as intravenous administration, transdermal administration, and topical administration. The dosage form suitable for oral administration includes tablet, capsule, granule, powder, troche, syrup, emulsion, and suspension; and the dosage form suitable for parenteral administration includes external preparation, suppository, injection, eye drop, eye ointment, plaster, gel, insertion agent, nasal preparation, and pulmonary preparation. These dosage forms can be prepared according to a method known in the technical field of pharmaceutical preparation.

Examples of the "pharmaceutically acceptable carrier" include various organic and inorganic carrier substances conventionally used as preparation materials; and include, for example, excipient, disintegrant, binder, fluidizer, lubricant and the like contained in solid preparations, and solvent, solubilizing agent, suspending agent, isotonicity agent, buffering agent, soothing agent and the like contained in liquid preparations. Where necessary, additives such as preservative, antioxidant, colorant, and sweetening agent can be further used.

Examples of the "excipient" include lactose, sucrose, D-mannitol, D-sorbitol, cornstarch, dextrin, microcrystalline cellulose, crystalline cellulose, carmellose, carmellose calcium, sodium carboxymethyl starch, low-substituted hydroxypropylcellulose, gum arabic and the like.

Examples of the "disintegrant" include carmellose, carmellose calcium, carmellose sodium, sodium carboxymethyl starch, croscarmellose sodium, crospovidone, low-substituted hydroxypropylcellulose, hydroxypropylmethylcellulose, crystalline cellulose and the like.

Examples of the "binder" include hydroxypropylcellulose, hydroxypropylmethylcellulose, povidone, crystalline cellulose, sucrose, dextrin, starch, gelatin, carmellose sodium, gum arabic and the like.

Examples of the "fluidizer" include light anhydrous silicic acid, magnesium stearate and the like.

Examples of the "lubricant" include magnesium stearate, calcium stearate, talc and the like.

Examples of the "solvent" include purified water, ethanol, propylene glycol, macrogol, sesame oil, corn oil, olive oil and the like.

Examples of the "solubilizing agents" include propylene glycol, D-mannitol, benzyl benzoate, ethanol, triethanolamine, sodium carbonate, sodium citrate and the like.

Examples of the "suspending agent" include benzalkonium chloride, carmellose, hydroxypropylcellulose, propylene glycol, povidone, methylcellulose, glycerol monostearate and the like.

Examples of the "isotonicity agent" include glucose, D-sorbitol, sodium chloride, D-mannitol and the like.

Examples of the "buffering agent" include sodium hydrogenphosphate, sodium acetate, sodium carbonate, sodium citrate and the like.

Examples of the "soothing agent" include benzyl alcohol and the like.

Examples of the "preservative" include ethyl parahydroxybenzoate, chlorobutanol, benzyl alcohol, sodium dehydroacetate, sorbic acid and the like.

Examples of the "antioxidant" include sodium sulfite, ascorbic acid and the like.

Examples of the "colorant" include food colors (e.g., Food Color Red No. 2 or 3, Food Color Yellow No. 4 or 5 etc.), β-carotene and the like.

Examples of the "sweetening agent" include saccharin sodium, dipotassium glycyrrhizinate, aspartame and the like.

The pharmaceutical composition of the present invention can be administered orally or parenterally (e.g., topical administration, rectal administration, intravenous administration etc.) to mammals other than human (e.g., mouse, rat, hamster, guinea pig, rabbit, cat, dog, swine, bovine, horse, sheep, monkey etc.) and also to human. The dose varies depending on the subject of administration, disease, symptom, dosage form, administration route and the like; but, for example, regarding oral administration to an adult patient (body weight about 60 kg), about 1 mg to 1 g of Compound A, which is an active ingredient, can be administered once or in several portions per day at any time before, after or with meals. The administration period is not particularly limited.

Compound A or a pharmaceutically acceptable salt thereof suppresses a tubulointerstitial fibrosis induced by local hypoxic condition, and a tubulointerstitial fibrosis induced by hyperglycemic condition; and it can thus be used as an active ingredient of an agent for treating or preventing a chronic kidney disease associated with diabetes, or diabetic nephropathy.

Furthermore, Compound A or a pharmaceutically acceptable salt thereof suppresses a tubulointerstitial fibrosis induced by local hypoxic condition; and it can thus also be used as an active ingredient of an agent for treating or preventing a chronic kidney disease associated with an immune system or inflammatory disease, or glomerulonephritis, and also a chronic kidney disease associated with hypertension, or nephrosclerosis.

The "immune system or inflammatory disease" means, among diseases caused by immune system abnormality or inflammation, a disease which consequently causes kidney injury. The "immune system or inflammatory disease" itself may be either chronic, subacute or acute, and may be either systemic or local. The "immune system or inflammatory disease" is preferably hereditary nephritis, IgA nephropathy, systemic lupus erythematosus, viral infection, bacterial infection, or parasitic disease.

Hyperglycemia is a symptom which is seen throughout from the preclinical stage to the end stage in both patients with diabetic nephropathy and patients with a chronic kidney disease associated with diabetes. Progression of renal injury caused by long-lasting hyperglycemia, particularly a tubulointerstitial fibrosis caused by damage due to hyperglycemia, promotes further renal impairment and leads the patients to end-stage renal disease.

Therefore, suppression of a tubulointerstitial fibrosis induced by hyperglycemic condition is meaningful in suppressing transition from diabetic nephropathy or a chronic kidney disease associated with diabetes to end-stage renal disease. In other words, in the present invention, treatment or prevention of diabetic nephropathy, and treatment or prevention of a chronic kidney disease associated with diabetes mean suppression of progression at any stage from the preclinical stage to the end stage, and preferably mean suppression or delay of the transition from diabetic nephropathy or a chronic kidney disease associated with diabetesto end-stage renal disease.

Furthermore, the local hypoxic condition in kidney tissue is involved in not only a tubulointerstitial fibrosis induced by hyperglycemia but also a tubulointerstitial fibrosis induced by each of immune system abnormality, inflammation and hypertension; and plays a partial role of the final common pathway. Suppression of a tubulointerstitial fibrosis induced by local hypoxic condition therefore can suppress the transition to end-stage renal disease even from the preclinical stage to the end stage of each of glomerulonephritis, nephrosclerosis, a chronic kidney disease associated with an immune system or inflammatory disease, and a chronic kidney disease associated with hypertension.

The "chronic kidney disease caused by hypoxia" means a chronic kidney disease which may progress to end-stage renal disease due to the fact that tubulointerstitial fibrosis has been induced by local hypoxic condition in the kidney tissue or there is a risk that tubulointerstitial fibrosis may be induced by local hypoxic condition in the kidney tissue. The aforementioned chronic kidney disease associated with an immune system or inflammatory disease, and a chronic kidney disease associated with hypertension are each included as one embodiment of the "chronic kidney disease caused by hypoxia". In addition, the chronic kidney disease associated with diabetes is also included in the "chronic kidney disease caused by hypoxia". The "chronic kidney disease caused by hypoxia" is preferably a chronic kidney disease associated with an immune system or inflammatory disease, or a chronic kidney disease associated with hypertension.

In the present specification, chronic kidney disease may be optionally indicated as CKD.

Thus, in the present specification, the "renal injury-related disease" refers to any disease which involves tubulointerstitial fibrosis and is targeted to the treatment or prevention in the present invention. The "renal injury-related disease" is preferably diabetic nephropathy, a chronic kidney disease associated with diabetes, glomerulonephritis, a chronic kidney disease associated with an immune system or inflammatory disease, nephrosclerosis, or a chronic kidney disease associated with hypertension.

In the present specification, "associated" means a condition where one disease coexists or is concomitant with another disease; and is not limited by whether a cause-and-effect relationship exists between the former and the latter, and even when such a relationship exists, it is not limited by whether the cause is the former or the latter.

In the present specification, the "treatment" includes improving symptom, avoiding increase in severity, maintaining remission, avoiding exacerbation, and also avoiding recurrence. In the present specification, "prevention" means suppressing the onset of symptom.

In the present specification, "an effective amount" means, for example, an amount of a medicament or a drug which produces a biological or medical reaction in the tissue, system, animal or human. In addition, "a therapeutically effective amount" means any amount which produces a treatment, healing, prevention or improvement in which the disease, disorder or side effect has been improved, or any amount which produces a decrease in the progression rate of the disease, compared with the corresponding subject which has not accepted such amount.

One embodiment of the present invention includes a method for treating or preventing a renal injury-related disease and a method for suppressing tubulointerstitial fibrosis comprising administering to a mammal a therapeutically effective amount of Compound A or a pharmaceutically acceptable salt thereof.

Mammal means mouse, rat, hamster, guinea pig, rabbit, cat, dog, pig, cattle, horse, sheep, monkey, human and the like; and preferably human.

Tubulointerstitial fibrosis includes a fibrosis induced by hyperglycemic condition and fibrosis induced by local hypoxic condition. Suppression of fibrosis or suppressing fibrosis means suppressing or delaying tubulointerstitial fibrosis.

One embodiment of the present invention includes a pharmaceutical composition for treating or preventing a renal injury-related disease, an agent for treating or preventing a renal injury-related disease, and an agent for suppressing tubulointerstitial fibrosis comprising Compound A or a pharmaceutically acceptable salt thereof. The definition and the like are as described above.

One embodiment of the present invention includes use of Compound A or a pharmaceutically acceptable salt thereof for the manufacture of a medicament for treating or preventing a renal injury-related disease, and use of Compound A or a pharmaceutically acceptable salt thereof for the manufacture of an agent for suppressing tubulointerstitial fibrosis. The definition and the like are as described above.

One embodiment of the present invention includes Compound A or a pharmaceutically acceptable salt thereof for use in treating or preventing a renal injury-related disease, and Compound A or a pharmaceutically acceptable salt thereof for use in suppression of tubulointerstitial fibrosis. The definition and the like are as described above.

### [Examples]

The present invention is explained in detail in the following by referring to Examples, but the present invention is not limited thereto.

### (Example 1)

### Experiment 1. Suppressive effect on tubulointerstitial fibrosis by dietary administration of Compound A in a rat model of diabetic nephropathy at 12 weeks of age for 28 weeks

Suppressive effect of Compound A on tubulointerstitial fibrosis was evaluated using a rat model of diabetic nephropathy. Male SHR/NDmcr-cp rat (Japan SLC, Inc.) was used as an experiment animal, and male WKY/Izm rat was used as a normal control (WKY group).

The above-mentioned two types of rats were purchased at 6 weeks of age. After confirming that SHR/NDmcr-cp rats have hyperglycemia, hypertension, and hyperlipidemia; Compound A (0.01 % diet) and vehicle (powdered diet) were administered to Compound A Group and Vehicle Group respectively from 12 weeks of age to 40 weeks of age.

At 40 weeks of age, the rats were exsanguinated under isoflurane anesthesia, kidney was collected and fixed in 10 % neutral buffered formalin, and then paraffin-embedded section was prepared. After deparaffinization, the section was stained with an iron hematoxylin solution for 8 minutes and then with a sirius red solution for 1 hour.

The iron hematoxylin solution was prepared at the time of use by mixing equal volume of Solution 1 (dissolving 1 g hematoxylin in 100 mL ethanol) and Solution 2 (dissolving 2 g ferric chloride (FeCl₃·6H₂O) and 1 mL of 25% hydrochloric acid in 95 mL distilled water). The sirius red solution was prepared by dissolving 0.5 g Direct Red 80 (Sigma-Aldrich Co.) in 500 mL picric acid saturated aqueous solution.

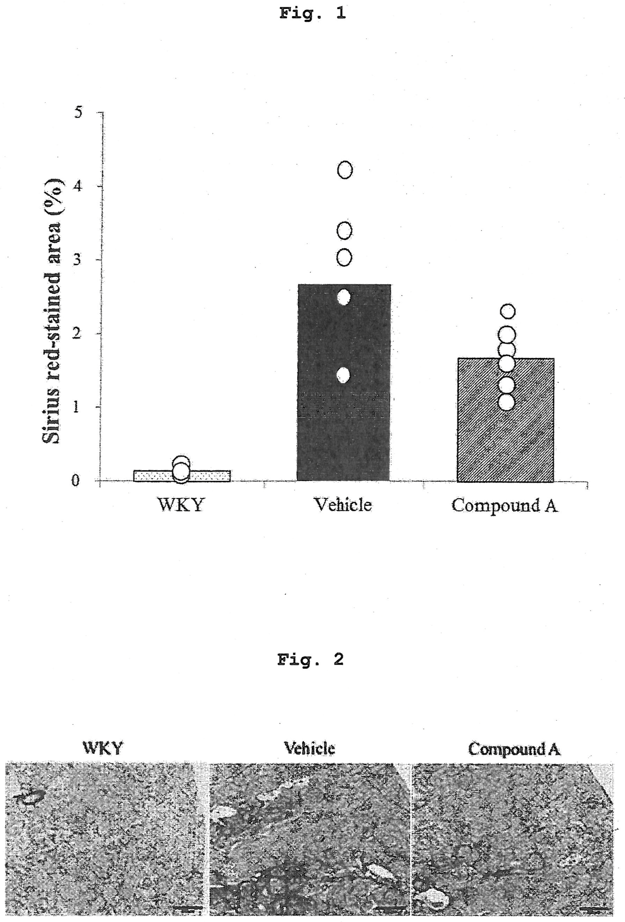

An image of the sirius red-stained section was captured with HS All-in-One Fluorescence Microscope (BZ-9000, KEYENCE CORPORATION), and the ratio (%) of sirius red-stained area relative to the whole kidney area was measured by a built-in image analysis function. An average area was calculated from the ratio of each section, and the results thereof are shown in Fig. 1. A representative stained image is shown in Fig. 2.

### (Example 2)

### Experiment 2. Suppressive effect on tubulointerstitial fibrosis by repeated oral administration of Compound A in a rat model of renal ischemia-reperfusion injury after 4 weeks from the ischemia-reperfusion injury, wherein Compound A was administered for 6 days starting from 3 days before the ischemia-reperfusion injury

Suppressive effect of Compound A on tubulointerstitial fibrosis was evaluated using a rat model of renal ischemia-reperfusion injury. Male SD rat at 6 weeks of age (CHARLES RIVER LABORATORIES JAPAN, INC.) was used as an experiment animal.

0.5 % (w/v) Methylcellulose (MC) was orally administered to Vehicle Group and Sham Group, and 0.2 mg/mL or 2 mg/mL Compound A suspended in 0.5 % (w/v) methylcellulose (MC) was orally administered to Compound A Group at a dose of 5 mL/kg once daily for 6 days from 3 days before the ischemia-reperfusion injury.

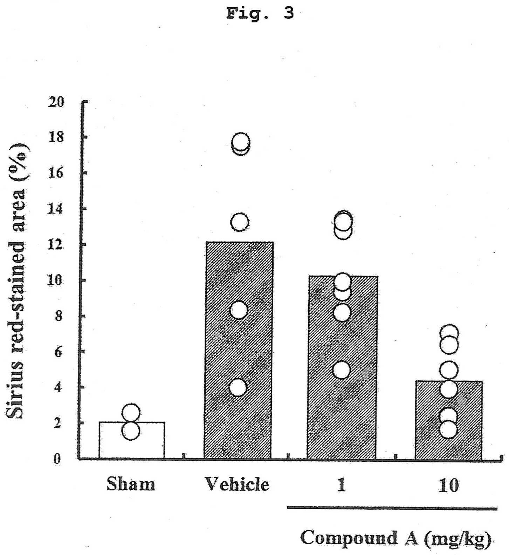

On one day before the ischemia-reperfusion injury, the right kidney was removed under isoflurane anesthesia. The ischemia-reperfusion injury was induced by clamping the left renal vein and artery for 45 minutes and then following reperfusion under isoflurane anesthesia, whereas such a surgery treatment was not performed for the Sham Group. 4 Weeks after the ischemia-reperfusion injury, the rats were exsanguinated under isoflurane anesthesia, kidney was collected and fixed in 10 % neutral buffered formalin, and then paraffin-embedded section was prepared. Staining and image analysis were performed by a similar method as described above. An average area was calculated from the stained ratio of each section, and the results thereof are shown in Fig. 3.

### [Industrial Applicability]

The present invention provides a novel pharmaceutical use of Compound A for a renal injury-related disease as a target disease.

This application is based on a patent application No. 2015-109207 filed in Japan, the contents of which are incorporated in full herein.

The present invention is summarized by the following items:
1. A method for treating or preventing diabetic nephropathy comprising administering to a mammal a therapeutically effective amount of a compound represented by the following chemical structural formula: 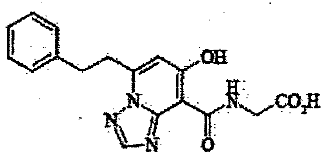 or a pharmaceutically acceptable salt thereof.
2. A method for treating or preventing a chronic kidney disease associated with diabetes comprising administering to a mammal a therapeutically effective amount of a compound represented by the following chemical structural formula: 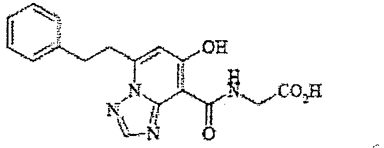 or a pharmaceutically acceptable salt thereof.
3. A method for treating or preventing a chronic kidney disease caused by hypoxia which is selected from the group consisting of a chronic kidney disease associated with an immune system or inflammatory disease and a chronic kidney disease associated with hypertension comprising administering to a mammal a therapeutically effective amount of a compound represented by the following chemical structural formula: 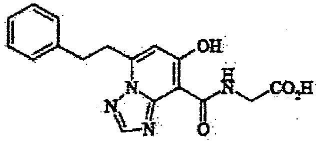 or a pharmaceutically acceptable salt thereof.
4. The method according to item 3 wherein the chronic kidney disease caused by hypoxia is a chronic kidney disease associated with an immune system or inflammatory disease.
5. The method according to item 3 wherein the chronic kidney disease caused by hypoxia is a chronic kidney disease associated with hypertension.
6. A method for treating or preventing glomerulonephritis comprising administering to a mammal a therapeutically effective amount of a compound represented by the following chemical structural formula: 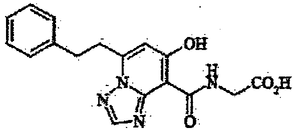 or a pharmaceutically acceptable salt thereof.
7. A method for treating or preventing nephrosclerosis comprising administering to a mammal a therapeutically effective amount of a compound represented by the following chemical structural formula: 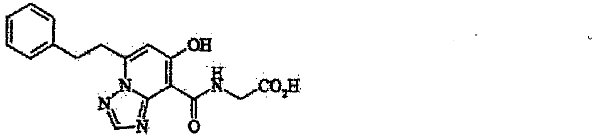 or a pharmaceutically acceptable salt thereof.
8. A method for suppressing tubulointerstitial fibrosis comprising administering to a mammal a therapeutically effective amount of a compound represented by the following chemical structural formula: 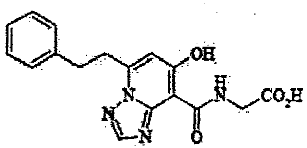 or a pharmaceutically acceptable salt thereof.
9. A pharmaceutical composition for treating or preventing a renal injury-related disease selected from the group consisting of diabetic nephropathy, a chronic kidney disease associated with diabetes, glomerulonephritis, a chronic kidney disease associated with an immune system or inflammatory disease, nephrosclerosis, and a chronic kidney disease associated with hypertension comprising a compound represented by the following chemical structural formula: 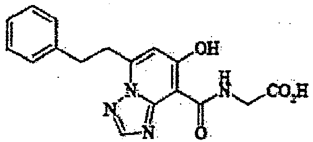 or a pharmaceutically acceptable salt thereof.
10. An agent for suppressing tubulointerstitial fibrosis comprising a compound represented by the following chemical structural formula: 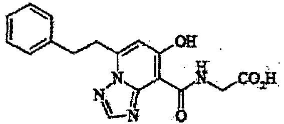 or a pharmaceutically acceptable salt thereof.
11. Use of a compound represented by the following chemical structural formula: 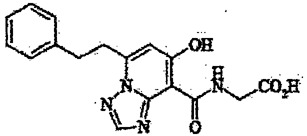 or a pharmaceutically acceptable salt thereof for the manufacture of a medicament for treating or preventing a renal injury-related disease selected from the group consisting of diabetic nephropathy, a chronic kidney disease associated with diabetes, glomerulonephritis, a chronic kidney disease associated with an immune system or inflammatory disease, nephrosclerosis, and a chronic kidney disease associated with hypertension.
12. Use of a compound represented by the following chemical structural formula: 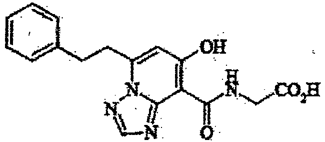 or a pharmaceutically acceptable salt thereof for the manufacture of an agent for suppressing tubulointerstitial fibrosis.

## Claims

1. A compound represented by the following chemical structural formula: 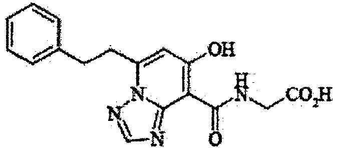 or a pharmaceutically acceptable salt thereof for use in treating or preventing diabetic nephropathy.

2. A compound represented by the following chemical structural formula: 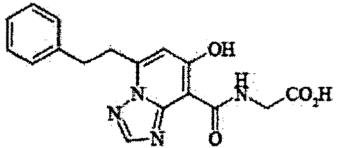 or a pharmaceutically acceptable salt thereof for use in treating or preventing a chronic kidney disease associated with diabetes.

3. A compound represented by the following chemical structural formula: 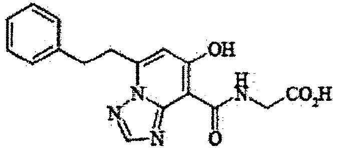 or a pharmaceutically acceptable salt thereof for use in treating or preventing a chronic kidney disease caused by hypoxia which is selected from the group consisting of a chronic kidney disease associated with an immune system or inflammatory disease and a chronic kidney disease associated with hypertension.

4. The compound for use according to claim 3 wherein the chronic kidney disease caused by hypoxia is a chronic kidney disease associated with an immune system or inflammatory disease.

5. The compound for use according to claim 3 wherein the chronic kidney disease caused by hypoxia is a chronic kidney disease associated with hypertension.

6. A compound represented by the following chemical structural formula: 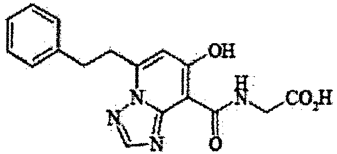 or a pharmaceutically acceptable salt thereof for use in treating or preventing glomerulonephritis.

7. A compound represented by the following chemical structural formula: 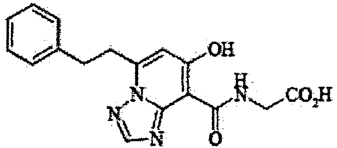 or a pharmaceutically acceptable salt thereof for use in treating or preventing nephrosclerosis.

8. A compound represented by the following chemical structural formula: 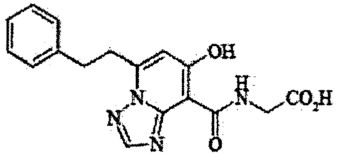 or a pharmaceutically acceptable salt thereof for use in suppressing tubulointerstitial fibrosis.

9. A pharmaceutical composition for use in treating or preventing a renal injury-related disease selected from the group consisting of diabetic nephropathy, a chronic kidney disease associated with diabetes, glomerulonephritis, a chronic kidney disease associated with an immune system or inflammatory disease, nephrosclerosis, and a chronic kidney disease associated with hypertension comprising a compound represented by the following chemical structural formula: 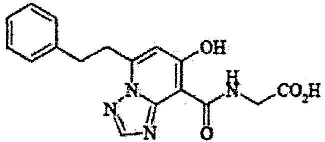 or a pharmaceutically acceptable salt thereof.

10. An agent for use in suppressing tubulointerstitial fibrosis comprising a compound represented by the following chemical structural formula: 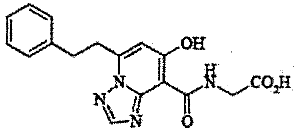 or a pharmaceutically acceptable salt thereof.
